# EUROPÄISCHE PATENTANMELDUNG

(11) **EP 1 256 337 A1**
(43) Veröffentlichungstag der Anmeldung: **13.11.2002**
(21) Anmeldenummer: 01111041.8
(22) Anmeldetag: 08.05.2001
(51) Int. Cl.: A61K 9/12, A61K 9/107, A61K 7/48

(54) **Verfahren und Zusammensetzungen zum Aufbringen eines Wirkstoffs auf ein Substrat**

(71) Anmelder: VESIFACT AG, 6342 Baar 2 (CH)
(72) Erfinder: Supersaxo, Andreas Werner, 6340 Baar (CH); Weder, Marc Antoine, 8803 Rüschlikon (CH); Weder, Hans Georg, 8803 Rüschlikon (CH)
(74) Vertreter: Hepp, Dieter

(57) **Zusammenfassung**

Die Erfindung betrifft ein Verfahren sowie Zusammensetzungen zum Aufbringen eines Wirkstoffs auf ein Substrat. Der Wirkstoff wird in eine Mikroemulsion eingebracht. Es wird Aufschäummittel und gegebenenfalls Schaumbildner derart zugegeben, dass sie nicht in die Teilchen der Mikroemulsion diffundieren und diese zerstören. Die Mikroemulsion wird auf dem Substrat aufgeschäumt. Bei der anschliessenden späteren Brechung des Schaums bildet sich ein hauchdünner Film. Der Wirkstoff kann über einen längeren Zeitraum kontinuierlich in das Substrat diffundieren.

## Beschreibung

Die Erfindung betrifft ein Verfahren zum Aufbringen eines Wirkstoffs auf ein Substrat, Zusammensetzungen zur Verwendung bei einem solchen Verfahren, ein Behälter zur Aufnahme der Zusammensetzung und Verwendung der Zusammensetzungen gemäss Oberbegriff der unabhängigen Patentansprüche.

Der Applikationsform von Wirkstoffen kommt heute eine besondere Bedeutung zu. Die Art und Weise wie ein Wirkstoff angeboten wird, entscheidet darüber, welche Menge eines Wirkstoffs in welcher Zeitspanne von einem Applikationsort an den Wirkungsort gelangt und damit auch welche Menge an Wirkstoff im Ausgangspräparat eingesetzt werden muss. Dies ist insbesondere dann relevant, wenn unerwünschte Nebenwirkungen eines Wirkstoffes, insbesondere im pharmazeutischen Bereich, verhindert werden sollen, oder wenn die Effektivität der Behandlung verbessert werden soll.

Mikroemulsionen sind ideale Formulierungen zur topischen, insbesondere dermalen Applikation von pharmazeutischen und kosmetischen Wirkstoffen. Sie verbessern die Bioverfügbarkeit der Wirkstoffe gegenüber herkömmlichen, aus dem Stand der Technik hinreichend bekannten Salben, Cremen, Lotionen und Gelen.

Mikroemulsionen weisen eine oder mehrere der folgenden Eigenschaften auf:
- Sie werden spontan gebildet, wenn ihre Komponenten miteinander in Kontakt gebracht werden;
- Sie sind praktisch nicht opaque, sondern transparent oder opaleszent, wenn sie unter einem optischen Mikroskop betrachtet werden; sie sind in ihrem nicht-gestörten Zustand optisch isotrop, obwohl sich bei einer Beobachtung beispielsweise unter Anwendung einer Röntgenstrahltechnik eine anisotrope Struktur feststellen lässt;
- Sie enthalten eine disperse oder stückige (Tröpfchen-) Phase, deren Teilchen eine Grösse von weniger als 200 nm haben, wovon ihre optische Transparenz herrührt.

Im Stand der Technik sind zur topischen Applikation, insbesondere zur dermalen Applikation im pharmazeutischen Bereich, vor allem Systeme gezeigt, bei denen die Mikroemulsion in Form eines wässerigen Sprays aufgetragen wird. So zeigt beispielsweise WO 97/21428 einen Cortisolspray basierend auf einer Mikroemulsion mit Wasser als Trägerflüssigkeit.
Aufgrund ihrer wässerigen Beschaffenheit weisen die aus dem Stand der Technik bekannten Sprays aber eine schlechte Oberflächenhaftung, insbesondere auf der Haut, auf. Besonders nachteilig ist dabei, dass die applizierte Mikroemulsion von der Oberfläche, insbesondere von der Haut, abfliessen kann. Eine reproduzierbare Applikation, respektive Dosierung wird dabei erschwert oder verunmöglicht.
Aufgabe der vorliegenden Erfindung ist es, die aus dem Stand der Technik bekannten Mängel zu vermeiden oder zu minimieren. Insbesondere soll eine Applikationsform zum Auftragen einer Mikroemulsion auf eine Oberfläche geschaffen werden, bei der ein gezieltes lokales Auftragen ermöglicht wird, welche eine reproduzierbare Applikation respektive Dosierung ermöglicht und welche insbesondere auch für topische, im besonderen für dermale Applikationen von Wirkstoffen im pharmazeutischen, respektive kosmetischen Bereich eingesetzt werden kann. Ausserdem soll die Bioverfügbarkeit verbessert werden.

Diese Aufgabe lässt sich überraschenderweise damit lösen, dass die Mikroemulsion in einer Schaumschicht auf das Substrat aufgebracht, insbesondere auf dem Substrat aufgeschäumt wird. Der Wirkstoff wird also in eine Mikroemulsion eingebracht und auf dem Substrat aufgeschäumt. Vor allem wird dabei die vorgängig mit Aufschäummittel und gegebenenfalls Schaumbildner versetzte Mikroemulsion aufgeschäumt ohne dass die Struktur der Teilchen der stückigen Phase, also die Partikel bzw. Nanopartikel der Mikroemulsion dabei in unerwünschter Weise verändert oder zerstört wird.

Wenn die Mikroemulsion in Form eines Schaums aus einem Behälter, insbesondere mit einer Sprühschaumvorrichtung, auf ein Substrat aufgebracht wird, lässt sich eine lokale Applikation an einer vorher definierten Stelle realisieren. Damit lässt sich nicht nur bezüglich Lokalität sondern auch hinsichtlich der aufgetragenen Menge eine reproduzierbare Applikation erreichen. Die erfindungsgemäss verwendeten Mikroemulsionen werden vorteilhaft vorgängig mit wenigstens einem Aufschäummittel und gegebenenfalls mit einem Schaumbildner versetzt, so dass sie selbstaufschäumend sind. Selbstaufschäumend im Sinne der Erfindung heisst, dass die Mikroemulsion erst beim Auftreffen auf der Oberfläche eines Substrates aufschäumt. Bei der anschliessenden spontanen Brechung des Schaums bildet sich ein hauchdünner Film. Während und nach dieser Filmbildung kann der in der Mikroemulsion enthaltene Wirkstoff über einen längeren Zeitraum kontinuierlich ins Substrat diffundieren. Besonders vorteilhaft ist dies, wenn die Mikroemulsion im pharmazeutischen oder kosmetischen Bereich topisch, insbesondere dermal appliziert wird. Gleichzeitig wird durch diesen Film die Transpiration der betroffenen Hautstelle reduziert. Als Folge davon nimmt die Hydratation der Haut zu, was die Wirkstoffaufnahme zusätzlich verbessern kann. Durch das erfindungsgemässe Verfahren wird zudem insbesondere eine schonende druckarme Applikation ermöglicht. Dies kann insbesondere bei der Behandlung von offenen Wunden oder Verbrennungen, beispielsweise mit Dexpanthenol, besonders vorteilhaft sein. Auch das Aufbringen von kosmetischen Wirkstoffen, wie beispielsweise von Q10 wird durch die Erfindung wesentlich verbessert.

Die mit Mikroemulsionen erreichbare hohe Bioverfügbarkeit der Wirkstoffe beruht darauf, dass der Wirkstoff in den Strukturen der Mikroemulsion gelöst vorliegt und damit in kleinsten Einheiten zur Aufnahme angeboten wird. Es ist deshalb erfindungswesentlich, dass die Struktur der Teilchen der stückigen Phase der Mikroemulsion durch das Aufschäumen nicht unerwünscht verändert oder zerstört wird. Dies lässt sich dadurch gewährleisten, dass Aufschäummittel und gegebenenfalls Schaumbildner im wesentlichen nicht in die Teilchen der stückigen Phase diffundieren. Bei der Auswahl von Aufschäummittel und Schaumbildner ist deshalb darauf zu achten, dass diese vorwiegend, vorzugsweise ausschliesslich in der wässerigen Phase der Mikroemulsion gelöst werden. Beim Aufschäumprozess wird so vor allem die wässerige Phase aufgeschäumt, während die Teilchen der stückigen Phase intakt bleiben.

Unter dem Begriff "im wesentlichen nicht in die Teilchen der stückigen Phase diffundieren" wird deshalb verstanden, dass die Teilchen der stückigen Phase nicht durch eindiffundiertes Aufschäummittel bei der Expansion des Aufschäummittels ebenfalls so expandieren, dass sie unerwünscht verändert oder zerstört werden. Bei der unerwünschten Veränderung oder Zerstörung der Teilchen der stückigen Phase bildet sich in der Regel eine Emulsion, d.h. die ursprünglichen Teilchen der stückigen Phase fusionieren zu wesentlich grösseren Teilchen, was unerwünscht ist. Dies lässt sich erfindungsgemäss mittels dynamischer Laserlicht-Streuung beispielsweise mit einem Nicomp 380 Particle Sizer überprüfen. Der dabei ermittelte mittlere Teilchendurchmesser der stückigen Phase soll auch nach dem Aufschäumen insbesondere maximal 200 nm, bevorzugterweise < 150 nm, noch bevorzugter < 100 nm betragen. Aufschäummittel wie Dymel 134a, Du Pont (1,1,1,2-Tetrafluorethan) diffundieren besonders leicht in die Teilchen der stückigen Phase, was vermieden werden soll.

Geeignete Aufschäummittel, die eine schonende Schaumbildung garantieren und die Teilchen der stückigen Phase nicht unerwünscht verändern oder zerstören sind flüssige Gase. Sie sollten bei Normaldruck (nach dem Ausbringen aus einem Schaumbehälter) bei einer Temperatur > -25°C, besonders vorteilhaft > 0°C von der flüssigen in die gasförmige Phase übergehen. Besonders bewährt haben sich Mischungen aus Isobutan und Isopentan. Art und Menge des Aufschäummittels sind jeweils auf die eingesetzte Mikroemulsion abzustimmen, so dass diese auch nach dem Aufschäumen strukturell intakt bleiben, d.h. dass ihre Teilchen der stückigen Phase nicht verändert oder zerstört werden.
Zum Auftragen einer Mikroemulsion in Form eines Schaums ist der Transfer der aufschäumbaren Mikroemulsion aus einem Behälter, insbesondere mit einer Sprühschaumvorrichtung, besonders vorteilhaft. Dazu muss die aufschäumbare Mikroemulsion in einem entsprechenden Behälter bereitgestellt werden.

Ein weiterer Gegenstand der Erfindung ist deshalb ein Verfahren zum Auftragen eines Wirkstoffs in Form einer selbstaufschäumenden Mikroemulsion auf ein Substrat.

Dieses Verfahren beinhaltet im wesentlichen folgende Schritte:
- Herstellen der Mikroemulsion enthaltend einen Wirkstoff.
- Zugabe von wenigstens einem Aufschäummittel und gegebenenfalls einem Schaumbildner, wobei Aufschäummittel und Schaumbildner im Hinblick auf die Zusammensetzung der Mikroemulsion so ausgewählt werden, dass sie im wesentlichen nicht in die Teilchen der stückigen Phase diffundieren.
- Abfüllung in einen Behälter, wobei der Behälter so als Schaumspender ausgebildet ist, dass die Mikroemulsion unter Ausbildung eines Schaums auf ein Substrat auftragbar ist.

Die Mikroemulsionen können dabei in bekannter Weise hergestellt werden, vorzugsweise durch Verdünnen eines Mikroemulsion-Prekonzentrates mit Wasser oder einer wässrigen Phase. Lipophile Wirkstoffe werden in das Mikroemulsion-Prekonzentrat eingearbeitet und durch Zugabe von Wasser oder einer wässerigen Phase in die Mikroemulsion übergeführt. Wasser lösliche Wirkstoffe werden vorab in Wasser oder einer wässrigen Phase gelöst. Durch Zugabe des Wirkstoff freien Mikroemulson- Prekonzentrats zu dieser Wirkstoff enthaltenden Wasserphase bildet sich spontan die entsprechende Mikroemulsion. Es ist auch möglich, einen oder mehrere lipophile Wirkstoffe in das Mikroemulsions-Prekonzentrat einzuarbeiten und zusätzlich einen oder mehrere wasserlösliche Wirkstoffe der Mikroemulsion bzw. der Wasserphase für die Mikroemulsion zuzugeben.

Als Alternative ist auch denkbar, dass ein Behälter ein Mikroemulsions-Prekonzentrat und eine wässerige Phase mit wenigstens einem Aufschäummittel in voneinander getrennten Kompartimenten enthält und eine Vermischung der beiden Phasen erst unmittelbar vor Applikation erfolgt. Das Aufschäummittel wird dabei erfindungsgemäss vorteilhaft nur in der wässerigen Phase vorgelegt.

Lipophile (ölige) Wirkstoffe, welche in das Prekonzentrat für die Mikroemulsion (vor Zugabe der wässrigen Phase) eingearbeitet sind, werden Teil der "stückigen Phase" der Mikroemulsion, das heisst, sie werden in die Nanopartikel der Mikroemulsion integriert. Lipophile Wirkstoffe, die auf diese Weise in Mikroemulsionen eingearbeitet sind, werden meist wesentlich besser resorbiert, als Wirkstoffe die z.B. in Emulsionen enthalten sind.

Völlig überraschend hat es sich erfindungsgemäss aber auch gezeigt, dass auch wasserlösliche Wirkstoffe, welche vorab in die zur Mikroemulsionsbildung benötigte Wasserphase eingearbeitet werden und daher im wesentlichen an in der Wasser Phase der Mikroemulsion gelöst und/oder an der Grenzfläche stückige Phase/Wasser Phase angelagert sind, besser resorbiert werden.

Besonders gut aufschäumbare und auftragbare Zusammensetzungen lassen sich erreichen, wenn ein Mikroemulsion-Prekonzentrat verwendet wird, dass enthält:
- (a1): ein Phospholipid
- (a2): ein natürliches oder ein synthetisches oder ein parti alsynthetisches Di- oder Triglycerid, *sowie optional* ein Wachs, einen Fettalkohol, Derviate dieser Stoffe sowie Mischungen dieser Stoffe
- (b): eine oberflächenaktive Komponente enthaltend ein Tensid vom Polyoxyethylentyp
Diese Mikroemulsion-Prekonzentrate können hergestellt werden, indem man die einzelnen Bestandteile, gegebenenfalls unter Erwärmen und Zuhilfenahme eines Lösungsvermittlers wie Ethanol, innig miteinander vermischt.

Die entsprechenden Mikroemulsionen sind erhältlich durch Verdünnen dieser Mikroemulsion-Prekonzentrate mit Wasser oder anderen wässrigen Flüssigkeiten. Dabei entstehen durch die im Prekonzentrat enthaltenen Komponenten (a1), (a2) und (b) spontan die Teilchen der stückigen Phase, deren Teilchengrösse kleiner als 200 nm, vorzugsweise kleiner als 150 nm, besonders bevorzugt kleiner als 100 nm ist. Ein weiterer Eintrag von Energie, insbesodere der Einsatz von hohen Scherkräften wird dabei nicht benötigt. Je nach der Menge des vorhandenen Wassers handelt es sich um W/O-Mikroemulsionen, um bikontinuierliche Mikroemulsionen oder O/W-Mikroemulsionen. Besonders bevorzugt im Rahmen der vorliegenden Erfindung sind dabei O/W-Mikroemulsionen.

Das Einbringen der Wirkstoffe in diese Mikroemulsionen erfolgt in bekannter Weise. Lipophile Wirkstoffe werden in das Mikroemulsion-Prekonzentrat und Wasser lösliche Wirkstoffe können vorteilhaft vorab in zur Mikroemulsionbildung benötigte Wasserphase eingearbeitet.
Vor allem für Mikroemulsionen, welche aus obigen Prekonzentraten enthaltend wenigstens (a1) ein Phospholipid, (a2) ein natürliches oder ein synthetisches oder ein partialsynthetisches Di- oder Triglycerid sowie optional, ein Wachs, ein Fettalkohol, Derviate dieser Stoffe sowie Mischungen dieser Stoffe und (b) eine oberflächenaktive Komponente enthaltend ein Tensid vom Polyoxyethylentyp hergestellt sind, haben sich als Aufschäummittel Isobutan und Isopentan bewährt. Besonders bevorzugt sind dabei Mischungen bestehend aus Isobutan und Isopentan.
Zur Unterstützung der Schaumbildung können die erfindungsgemäss eingesetzten Mikroemulsionen im weiteren wenigstens einen Schaumbildner enthalten. Dieser muss ebenfalls so ausgewählt sein, dass er im wesentlichen nicht in die Teilchen der stückigen Phase diffundiert sondern bevorzugt in der wässerigen Phase löslich ist. Besonders bewährt haben sich dabei Schaumbildner ausgewählt aus der Gruppe der Betaine, Sulfosuccinate oder Sarcosin-Derivate. Diese sind beispielsweise unter den Bezeichnungen Betain 810 (Capryl/Capramidopropyl Betaine), Crodasinic LS35 (Aqua and Sodium Lauroyl Sarcosinate) und Rewopol SB FA 30 ((Disodium Lauroyl Sarcosinate) bekannt und im Handel erhältlich. Besonders bevorzugt ist dabei Rewopol SB FA 30.

Vorzugsweise enthält das erfindungsgemäss eingesetzte Mikroemulsion-Prekonzentrat als Komponente (a1) ein Phospholipid, ein hydriertes oder teilhydriertes Phospholipid, ein Lysophospholipid, ein Ceramid oder Mischungen aus diesen Verbindungen 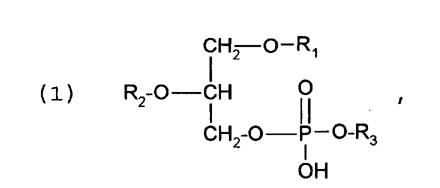 worin
- R₁: C₁₀-C₂₀-Acyl;
- R₂: Wasserstoff oder C₁₀-C₂₀-Acyl
- R₃: Wasserstoff, 2-Trimethylamino-1-ethyl, 2-Amino-1-ethyl,
nicht substituiertes oder durch eine oder mehrere Carboxy-, Hydroxy- oder Amino-Gruppen substituiertes C₁-C₅-Alkyl; die Inositol- oder die Glycerylgruppe;
bedeuten; oder Salze dieser Verbindungen.

C₁₀-C₂₀-Acyl ist vorzugsweise geradkettiges C₁₀-C₂₀-Alkanoyl mit einer geraden Anzahl an C-Atomen und geradkettiges C₁₀-C₂₀-Alkenoyl mit einer Doppelbindung und einer geraden Anzahl an C-Atomen.
Geradkettiges C₁₀-C₂₀-Alkanoyl mit einer geraden Anzahl an C-Atomen sind beispielsweise n-Dodecanoyl, n-Tetradecanoyl, n-Hexadecanoyl oder n-Octadecanoyl.
Geradkettiges C₁₀-C₂₀-Alkenoyl mit einer Doppelbindung und einer geraden Anzahl an C-Atomen sind beispielsweise 6-cis- oder-6-trans-, 9-cis- oder 9-trans-Dodecenoyl, -Tetradecenoyl,-Hexadecenoyl, -Octadecenoyl oder -Icosenoyl, insbesondere 9-cis-Octa-decenoyl (Oleoyl), ferner 9,12-cis-Octadecadienoyl oder 9,12,15-cis-octadecatrienoyl.

Ein Phospholipid der Formel (1), worin R₃ 2-Trimethylamino-1-ethyl bedeutet, wird mit dem Trivialnamen Lecithin und ein Phospholipid der Formel (1), worin R₃ 2-Amino-1-ethyl bedeutet, mit dem Trivialnamen Kephalin bezeichnet. Geeignet sind beispielsweise natürlich vorkommendes Kephalin oder Lecithin, z.B. Kephalin oder Lecithin aus Sojabohnen oder Hühnerei mit verschiedenen oder identischen Acylgruppen oder Mischungen davon.

Das Phospholipid der Formel (1) kann aber auch synthetischen Ursprungs sein. Unter dem Begriff synthetisches Phospholipid definiert man Phospholipide, welche bezüglich R₁ und R₂ eine einheitliche Zusammensetzung haben. Solche synthetischen Phospholipide sind vorzugsweise die weiter oben definierten Lecithine und Kephaline, deren Acylgruppen R₁ und R₂ eine definierte Struktur haben und von einer definierten Fettsäure mit einem Reinheitsgrad höher als ca. 95% abgeleitet sind. R₁ und R₂ können gleich oder verschieden und ungesättigt oder gesättigt sein. Bevorzugt ist R₁ gesättigt, z.B. n-Hexadecanoyl, und R₂ ungesättigt, z.B. 9-cis-Octadecenoyl (Oleoyl).

Der Begriff "natürlich vorkommendes" Phospholipid definiert Phospholipide, welche bezüglich R₁ und R₂ keine einheitliche Zusammensetzung haben. Solche natürlichen Phospholipide sind ebenfalls Lecithine und Kephaline, deren Acylgruppen R₁ und R₂ von natürlich vorkommenden Fettsäuregemischen abgeleitet sind.

Die Forderung "im wesentlichen reines" Phospholipid der Formel (1) definiert einen Reinheitsgrad von mehr als 90 Gew.-%, vorzugsweise mehr als 95 Gew.-% des Phospholipids der Formel (1), welcher anhand geeigneter Bestimmungsmethoden, z.B. papier- oder dünnschichtchromatographisch, mit HPLC oder enzymatischem Farbtest, nachweisbar ist.

In einem Phospholipid der Formel (1) ist R₃ mit der Bedeutung C₁-C₄-Alkyl beispielsweise Methyl oder Ethyl. Die Bedeutung Methyl ist bevorzugt. R₃ mit den Bedeutungen durch eine oder mehrere Carboxy-, Hydroxy- oder Aminogruppen substituiertes C₁-C₅-Alkyl sind beispielsweise 2-Hydroxyethyl, 2,3-Dihydroxy-n-propyl, Carboxymethyl, 1- oder 2-Carboxyethyl, Dicarboxymethyl, 2-Carboxy-2-hydroxyethyl oder 3-Carboxy-2,3-dihydroxy-n-propyl, 3-Amino-3-carboxy-n-propyl oder 2-Amino-2-carboxy-n-propyl, vorzugsweise 2-Amino-2-carboxyethyl.

Phospholipide der Formel (1) mit diesen Gruppen können in Salzform, z.B. als Natrium- oder Kaliumsalz, vorliegen.

Phospholipide der Formel (1), worin R₃ die Inositol- oder die Glycerylgruppe bedeutet, sind unter den Bezeichnungen Phosphatidylinositol und Phosphatidylglycerol bekannt.

Für die Acylreste in den Phospholipiden der Formel (1) sind auch die in Klammern angegebenen Bezeichnungen gebräuchlich: 9-cis-Dodecenoyl (Lauroleoyl), 9-Cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 9,12-cis-Octadecadienoyl (Linoleoyl), 9,12,15-cis-Octadecatrienoyl (Linolenoyl), 11-cis-Octadecenoyl (Vaccenoyl), 9-cis-Icosenoyl (Gadoleoyl), 5,8,11,14-cis-Eicosatetraenoyl (Arachidonoyl), n-Dodecanoyl, (Lauroyl), n-Tetradecanoyl (Myristoyl), n-Hexadecanoyl (Palmitoyl), n-Octadecanoyl (Stearoyl), n-Icosanoyl (Arachidoyl), n-Docosanoyl (Behenoyl), n-Tetracosanoyl (Lignoceroyl).

Ein Salz des Phospholipids der Formel (1) ist vorzugsweise pharmazeutisch annehmbar. Salze definieren sich durch die Existenz von salzbildenden Gruppen im Substituenten R₃ sowie durch die freie Hydroxygruppe am Phosphoratom. Möglich ist ebenfalls die Bildung von inneren Salzen. Bevorzugt sind Alkalimetallsalze, insbesondere Natriumsalze.

In einer besonders bevorzugten Ausführungsform verwendet man gereinigtes Lecithin aus Sojabohnen der Qualität LIPOID S 100 oder S 75 oder ein Lecithin definiert in der Monographie USP23/NF 18.

Die Komponente (a1) wird vorzugsweise in einer Konzentration von ca. 0,1 bis 30 Gew.-%, bezogen auf das Gesamtgewicht der Komponenten (a1), (a2) und (b), eingesetzt.

Vorzugsweise enthält das erfindungsgemäss eingesetzte Mikroemulsion-Prekonzentrat als Komponente (a2) ein natürliches oder ein synthetisches oder ein partialsynthetisches Di- oder Triglycerid, optional ein Wachs, ein Fettalkohol, Derviate dieser Stoffe sowie Mischungen dieser Stoffe. Bevorzugt sind mittelkettige Fettsäuretriglyceride vom Capryl-/Caprinsäure-Triglyceride Typ. Dazu gehören insbesondere die unter den Warenbezeichnungen MIGLYOL, MYRITOL, CAPTEX, CAP-MUL MCT, NEOBEE M5 und MAZOL 1400 bekannten und im Handel erhältlichen Zusammensetzungen (Fiedler, Lexikon der Hilfsstoffe, 3. Auflage, Seiten 808 bis 809 und 834, 1989). Besonders bevorzugt ist dabei MIGLYOL 812, ein fraktioniertes Kokosnussöl, das Triglyceride von Caprylsäure und Caprinsäure enthält.

Die Komponente (a2) ist im erfindungsgemäss eingesetzten Mikroemulsion-Prekonzentrat in einer Konzentration von 0,1 bis 80 Gew.-%, bevorzugt in einer Konzentration von 10 bis 50 Gew.-%, bezogen auf das Gesamtgewicht der Komponenten (al), (a2) und (b) vorhanden.

Bei Komponente (b) des erfindungsgemäss eingestetzen Mikroemulsion-Prekonzentrate, der oberflächenaktiven Komponente, enthaltend ein Tensid vom Polyoxyethylen-Typ, kann es sich um ein hydrophiles und/oder um ein lipophiles Tensid oder um Gemische solcher Tenside handeln. Nähere Angaben zu den nachfolgend aufgeführten und im Handel erhältlichen Produkten finden sich in "Fiedler, Lexikon der Hilfsstoffe, 3. Auflage, 1989".

Beispiele für solche Tenside sind:
- Polyoxyethylenglykolierte natürliche oder hydrierte Pflanzenöle. Besonders geeignet sind CREMOPHOR, insbesondere CREMOPHOR RH 40, CREMOPHOR RH 60 und CREMOPHOR EL. Ferner NIKKOL, insbesondere NIKKOL HCO-60.
- Polyoxyethylensorbitanfettsäureester, wie sie beispielsweise unter der Bezeichnung TWEEN erhältlich sind, insbesondere TWEEN 80.
- Polyoxyethylenfettsäureester, beispielsweise MYRJ, insbesondere MYRJ 52 oder CETIOL HE.
- Copolymerisate von Polyoxyethylen und Polyoxypropylen wie PLURONIC, insbesondere PLURONIC F68, und EMKALYX
- Blockcopolymerisate von Polyoxyethylen und Polyoxypropylen, beispielsweise POLOXAMER, insbesondere POLOXAMER 188.
- Polyethoxylierte Vitamin E Derivate, insbesondere VITAMIN E TPGS (d-Alpha Tocoperyl Polyethylene Glycol 1000 Succinate, Eastman).
- Polyethoxylierte Hydroxyfettsäureester, insbesondere SOLUTOL HS 15 (Polyoxyethylen-660-Hydroxystearat, BASF).
- Umesterungsprodukte von natürlichen Pflanzenölglyceriden und Polyethylenpolyolen. Dazu gehören LABRAFIL, insbesondere LABRAFIL M 1944 CS und LABRAFIL M 2130 CS.
- Ethylenoxidaddukte von Sterolen und Derivaten davon, beispielweise GENEROL, insbesondere GENEROL 122 E5, 122 E10 und 122 E25.

Die erfindungsgemäss eingesetzten Mikroemulsion-Prekonzentrate umfassen sowohl Systeme, die ein einzelnes oberflächenaktives Mittel enthalten, als auch Systeme, die ein Gemisch von zwei oder mehreren oberflächenaktiven Mitteln enthalten, z.B. TWEEN 80 + CREMOPHOR RH 40, TWEEN 80 + CREMOPHOR RH 40 + VITAMIN E TPGS etc.

Vorzugsweise verwendet man eine oberflächenaktive Komponente, welche einen Polyoxyethylensorbitanfettsäureester, ein polyoxyethylenglykoliertes natürliches oder hydriertes Pflanzenöl oder Mischungen davon enthält.

Bevorzugterweise ist die oberflächenaktive Komponente (b) in den erfindungsgemäss verwendeten Mikroemulsion-Prekonzentraten in einer Menge von 20 bis 80 Gewichtsprozent bezogen auf das Gesamtgewicht des Prekonzentrates vorhanden.

Die erfindungsgemäss eingesetzten Mikroemulsion-Prekonzentrate und Mikroemulsionen können auch noch weitere Stoffe enthalten, wie z.B. Antioxidantien, Riech- und/oder Geschmacksstoffe, Farbstoffe, Konservierungsmittel, Hautpenetrationsverbesserer etc.

Die mit dem beschriebenen Verfahren hergestellte Mikroemulsionen eignen sich als Vehikel für Wirkstoffe. Diese können namentlich pharmazeutische, kosmetische oder biotechnologische Wirkstoffe oder generell Stoffe sein, die für einen bestimmten technischen Zweck eingesetzt werden.

Ein Beispiel eines pharmazeutischen Wirkstoffes, welcher mit den aufschäumbaren Mikroemulsionen besonders vorteilhaft appliziert werden kann, ist Dexpanthenol. Erfindungsgemäss können grundsätzlich alle pharmazeutischen Wirkstoffe, welche topisch, insbesondere dermal, appliziert werden können, mit den aufschäumbaren Mikroemulsionen aufgetragen werden. Vorzugsweise werden Wirkstoffe aus folgenden therapeutischen Klassen verwendet:
Antiallergika, Antirheumatika/Entzündungshemmer, Lokalanästhetika, Venenmittel/Vasoprotektiva, Wundheilmittel, Hämorrhoidenmittel, Dermokortikoide, Antiseptika, Antibiotika, Antimykotika, antivirale Mittel, Antiparasitaria, Wirkstoffe zur Behandlung von Akne, Ekzemen, Psoriasis und Rosazea, Antipruriginosa, Antihidrotika, Adstringentia, Anitfibrotika, Desinfizientia, Wirkstoffe zur Behandlung von Hyperpigmentierung, Warzen und Hühneraugen, Wirkstoffe für Haar und Nägel, Hautschutz- und
Pflegemittel, UV-Strahlenschutzmittel und Vitamine. Wirkstoffe der oben erwähnten therapeutischen Klassen können dem Arzneimittel Kompendium der Schweiz 2001, Documed AG, CH-4010 Basel, Herausgeber: Jürg Morant und Hans Ruppanner, entnommen werden. Selbstverständlich können, falls therapeutisch gewünscht, auch Mischungen dieser Stoffe verwendet werden.

Weitere Wirkstoffe im Sinne der vorliegenden Erfindung sind Wirkstoffe, die sich für eine transdermale Therapie eigenen wie z.B. Hormone, Scopolamin und Nicotin .

Kosmetische Wirkstoffe im Sinne der vorliegenden Erfindung sind beispielsweise Aminosäuren, Peptide, Proteine oder deren Hydrolysate, Coenzyme, Flavonoide, UV-Filter und Vitamine.

Beispiele biotechnologischer Wirkstoffe sind Peptide und Proteine, insbesondere rekombinante Proteine. Beispiele von natürlichen und rekombinanten Proteinen sind Wachstumsfaktoren wie Epidermal Growth Factor (EGF), Keratinocyte Growth Factor (KGF), Acidic und Basic Fibroblast Growth Factor (aFGF und bFGF), Insulin-Like Growth Factor-I und II (IGF-I und IGF-II), Nerve Growth Factor (NGF), Platelet-Derived Growth Factors (PDGFs), Stem Cell Factors und Transforming Growth Factors; Chemotaktische Faktoren wie Macrophage/ Monocyte Chemotactic and Activating Factor (MCAF); Colony-Stimulating Faktoren wie Granulocyte-Macrophage Colony-Stimulating Factor (GM-CSF) und Granulocyte-Colony-Stimulating Factor (G-CSF); Interferone und Interleukine; Zelladhäsions- und Extracelluläre Matrix Proteine wie Kollagene, Fibronektine, Integrine, Laminine, Merosine, Proteoglykane, RGD-Adhäsionspeptide, Tenascine, Thrombospondine und Vitronektine und Enzyme wie Collagenase, Dispase und Trypsin. Weitere Beispiele von Proteinen sind Insulin, Albumin und Transferrin.

Technische Wirkstoffe im Sinne der Erfindung können alle Stoffe sein, die zum Erreichen eines bestimmten technischen Zwecks in die Mikroemulsion eingearbeitet werden. Beispiele hierfür sind Schmierstoffe oder Markerstoffe, insbesondere Pigmente.

Zur Applikation der neuen Zusammensetzung und zur Durchführung des Verfahrens gemäss der vorliegenden Erfindung eignen sich Behälter in der Form von Sprühschaumspendern.

Besonders geeignete Behälter im Sinne der Erfindung sind Aerosolspraydosen, insbesondere ein- und zweikammerige Aerosolspraydosen. Diese ermöglichen eine Lagerung der mit Aufschäummittel und gegebenenfalls Schaumbildner versetzten Mikroemulsion. Die zum Ausbringen der Mikroemulsion eingesetzten Treibmittel sind so zu wählen, dass sie die Teilchen der stückigen Phase nicht unerwünscht verändern oder zerstören. Geeignete Treibmittel sind Flüssiggase oder komprimierte Gase, insbesondere komprimierter Stickstoff.

Weitere besonders gut aufschäumbare und auftragbare Zusammensetzungen lassen sich erreichen, wenn ein Mikroemulsion-Prekonzentrat verwendet wird, dass enthält:
(a) eine Mischung bestehend aus einem mittelkettigen Triglycerid und einer Omega-9-Fettsäure und/oder einer Omega-6-Fettsäure
(b) eine oberflächenaktive Komponente enthaltend ein Tensid vom Polyoxyethylentyp

Das Mikroemulsion-Prekonzentrat wird mit Wasser oder einem wässerigen Medium gemischt, wobei spontan die Mikroemulsion gebildet wird. Das Einbringen der Wirkstoffe in die Mikroemulsion erfolgt in bekannter Weise. Lipophile Wirkstoffe werden in das Mikroemulsion-Prekonzentrat und Wasser lösliche Wirkstoffe werden vorteilhaft vorab in zur Mikroemulsionbildung benötigte Wasserphase eingearbeitet.

Aufschäummittel und gegebenenfalls Schaumbildner werden so ausgewählt, dass sie Teilchen der stückigen Phase nicht unerwünscht verändern oder zerstören.
Die zur Herstellung dieser Mikroemulsionen verwendeten Mikroemulsion-Prekonzentrate können hergestellt werden, indem man die einzelnen Bestandteile, gegebenenfalls unter Erwärmen, innig miteinander vermischt und, wie vorstehend beschrieben, mit Wasser verdünnt.
Das Mikroemulsion-Prekonzentrat enthält als Komponente (a) Mischungen aus einem mittelkettigen Fettsäuretriglycerid, zweckmässigerweise einem Fettsäuretriglycerid, in welchem die Fettsäurereste 4 bis 18, vorzugsweise 6 bis 18 C-Atome aufweisen, und einer Omega-9- und/oder einer Omega-6-Fettsäure.
Bevorzugte mittelkettige Fettsäuretriglyceride sind wie vorstehend beschrieben. Besonders bevorzugt ist dabei MIGLYOL 812, ein fraktioniertes Kokosnussöl, das Triglyceride von Caprylsäure und Caprinsäure enthält.

Geeignete Omega-9-Fettsäuren sind hauptsächlich solche mit 12-24, insbesondere 16-24, vorzugsweise 18-22 C-Atomen, beispielsweise Oelsäure und Eicosatriensäure. Besonders bevorzugt ist die Oelsäure.

Geeignete Omega-6-Fettsäuren sind hauptsächlich solche mit 12-24, insbesondere 16-24, vorzugsweise 18-22 C-Atomen, beispielsweise Linolsäure, gamma-Linolensäure, dihommo-gamma-Linolensäure und Arachidonsäure. Besonders bevorzugt ist die Linolsäure.

In einer besonders bevorzugten Ausführungsform verwendet man als Komponente (a) eine Mischung bestehend aus einem Capryl-/Caprinsäure-Triglycerid, Oelsäure und/oder Linolsäure.

Bevorzugterweise ist die Komponente (a) in den erfindungsgemäss verwendeten Mikroemulsion-Prekonzentraten in einer Menge von 20 bis 70 Gewichtsprozent bezogen auf das Gesamtgewicht des Prekonzentrates vorhanden.

Das Mengenverhältnis von Omega-9-Fettsäure und/oder Omega-6-Fettsäure zum mittelkettigen Triglycerid liegt bevorzugt im Bereich 1:1 bis 1:200, noch bevorzugter im Bereich 1:2 bis 1:20.

Bei Komponente (b) der Mikroemulsion-Prekonzentrate, der oberflächenaktiven Komponente enthaltend ein Tensid vom Polyoxyethylen-Typ, kann es sich um ein hydrophiles und/oder um ein lipophiles Tensid oder um Gemische solcher Tenside handeln, wie vorstehend beschrieben wurde.

Beispiele solcher Tenside sind vorstehend beschrieben.

Die erfindungsgemäss eingesetzten Mikroemulsion-Prekonzentrate umfassen sowohl Systeme, die ein einzelnes oberflächenaktives Mittel enthalten, als auch Systeme, die ein Gemisch von zwei oder mehreren oberflächenaktiven Mitteln enthalten, z.B. TWEEN 80 + CREMOPHOR RH 40, TWEEN 80 + CREMOPHOR RH 40 + VITAMIN E TPGS etc.
Die Mikroemulsion-Prekonzentrate und Mikroemulsionen können auch noch weitere Stoffe enthalten, wie z.B. Antioxidantien, Riech- und/oder Geschmacksstoffe, Farbstoffe, Konservierungsmittel, Hautpenetrationsverbesserer etc. Die mit dem beschriebenen Verfahren hergestellte Mikroemulsionen eignen sich als Vehikel für Wirkstoffe, wie vorstehend beschrieben.

Nachfolgend wird die vorliegende Erfindung anhand von Ausführungsbeispielen näher erläutert.

Die Beispiele 1-3 zeigen die Herstellung von Mikroemulsion-Prekonzentraten, Mikroemulsionen und selbstaufschäumenden Mikroemulsionen.

**Beispiel 1: Herstellung von Mikroemulsion-Prekonzentraten**

| Beispiel 1.1: Vitamin E Acetat 2% Mikroemulsion-Prekonzentrat | |
|---|---|
| Lecithin | 11.00% |
| Miglyol 812 | 48.00% |
| Tween 80 | 34.00% |
| Propylenglykol | 5.00% |
| Vitamin E Acetat | 2.00% |

Herstellung: Lecithin wird in Miglyol 812 gelöst. Zur erhaltenen Lösung fügt man Polysorbat 80, Propylenglykol und Vitamin E Acetat hinzu und rührt, bis man eine homogene, klare Flüssigkeit erhält.

| Beispiel 1.2: Coenzym Q10 1% Mikroemulsion-Prekonzentrat | |
|---|---|
| Lecithin | 11.00% |
| Miglyol 812 | 49.00% |
| Tween 80 | 34.00% |
| Propylenglykol | 5.00% |
| Coenzyme Q10 | 1.00% |

Herstellung: Lecithin wird in Miglyol 812 gelöst. Zur erhaltenen Lösung fügt man Polysorbat 80, Propylenglykol und Coenzym Q10 hinzu und rührt, gegebenenfalls unter leichtem Erwärmen, bis man eine homogene, klare Flüssigkeit erhält.

### Beispiel 2: Herstellung von Mikroemulsionen

Die in den Beispielen 1.1 und 1.2 beschriebenen Mikroemulsion-Prekonzentrate dienen nachfolgend als Basis zur Herstellung von Mikroemulsionen.

| Beispiel 2.1: Vitamin E Acetat 0.1% Mikroemulsion | |
|---|---|
| Mikroemulsion-Prekonzentrat, Beispiel 1.1 | 5.00 % |
| 10 mM Phosphatpuffer, pH 6 | ad 100,00 % |

Herstellung: Die Wasserphase wird unter Rühren (z.B. Magnetrührerwerk) bei 50°C vorgelegt. Das flüssige Mikroemulsion-Prekonzentrat, Beispiel 1.1, wird unter Rühren (z.B. mit einem Magnetrührwerk) der Wasserphase zugegeben.

| Beispiel 2.2: Coenzym Q10 0.05% Mikroemulsion | |
|---|---|
| Mikroemulsion-Prekonzentrat, Beispiel 1.2 | 5.00 % |
| 10 mM Phosphatpuffer, pH 6 | ad 100,00 % |

Herstellung: Die Wasserphase wird unter Rühren (z.B. Magnetrührerwerk) bei 50°C vorgelegt. Das flüssige Mikroemulsion-Prekonzentrat, Beispiel 1.2, wird unter Rühren (z.B. mit einem Magnetrührwerk) der Wasserphase zugegeben.

### Beispiel 3: Herstellung von selbstaufschäumenden Mikroemulsionen

Die in den Beispielen 2.1 und 2.2 beschriebenen Mikroemulsionen dienen nachfolgend als Basis zur Herstellung von selbstaufschäumenden Mikroemulsionen.

**Beispiel 3.1**

| Selbstaufschäumende Vitamin E Acetat 0.1% O/W Mikroemulsion | |
|---|---|
| Dexpanthenol 5% W/O Mikroemulsion, Beispiel 2.1 | 96.0% |
| Rewopol SB FA 30 | 4.0% |
| Isobutan/Isopentan | q.s. |
| Komprimierter Stickstoff | q.s. |

Herstellung: Die Vitamin E Acetat 0.1% O/W Mikroemulsion, Beispiel 2.1, wird mit Rewopol SB FA 30 (Schaumbildner) versetzt, in Einkammer-Aerosoldosen abgefüllt und mit Isobutan/Isopentan (Aufschäummittel) sowie komprimiertem Stickstoff (Treibmittel) versetzt.

Aus nachfolgender Tabelle ist ersichtlich, dass die Teilchengrösse obiger Mikroemulsion vor und nach dem Aufschäumen vergleichbar ist.

### Tabelle 1:

Selbstaufschäumende Vitamin E Acetat 0.1% O/W Mikroemulsion: Teilchengrösse der nicht aufgeschäumten und aufgeschäumten O/W Mikroemulsion

| **Präparat** | **Teilchendurchmesser**^{**1**} **[nm]** |
|---|---|
| Nicht aufgeschäumte O/W Mikroemulsion (enthält kein Aufschäummittel) | 28.3 ± 13.6 |
| Aufgeschäumte und wieder verflüssigte O/W Mikroemulsion | 32.2 ± 16.3 |

| | |
|---|---|
| ¹Die Teilchendurchmesser und die Teilchengrössenverteilungen wurden mittels dynamischer Laserlicht-Streuung gemessen (Gerät: Nicomp 380 Submicron Particle Sizer, Auswertung: Volume Weighting). | |

**Beispiel 3.2**

| Selbstaufschäumende Coenzym Q10 0.05% O/W Mikroemulsion | |
|---|---|
| Coenzym Q10 0.05% O/W Mikroemulsion, Beispiel 2.2 | 93.0% |
| Rewopol SB FA 30 | 4.0% |
| Isobutan/Isopentan | q.s. |

Die Coenzym Q10 0.05% O/W Mikroemulsion, Beispiel 2.2, wird mit Rewopol SB FA 30 (Schaumbildner) und Isobutan/Isopentan (Aufschäummittel) versetzt und in Zweikammer-Aerosoldosen, deren Aussenkammern mit dem Treibmittel n-Butan vorbegast wurden, abgefüllt.

Aus nachfolgender Tabelle ist ersichtlich, dass die Teilchengrösse obiger Mikroemulsion vor und nach dem Aufschäumen vergleichbar ist.

### Tabelle 2:

Selbstaufschäumende Coenzym Q10 0.05% O/W Mikroemulsion: Teilchengrösse der nicht aufgeschäumten und aufgeschäumten O/W Mikroemulsion

| **Präparat** | **Teilchendurchmesser**^{**2**} **[nm]** |
|---|---|
| Nicht aufgeschäumte O/W Mikroemulsion (enthält kein Aufschäummittel) | 36.6 ± 17.8 |
| Aufgeschäumte und wieder verflüssigte O/W Mikroemulsion | 41.2 ± 21.1 |

| | |
|---|---|
| ² Die Teilchendurchmesser und die Teilchengrössenverteilung wurde mittels dynamischer Laserlicht-Streuung gemessen (Gerät: Nicomp 380 Submicron Particle Sizer, Auswertung: Volume Weighting). | |

## Patentansprüche

1. Zusammensetzung zum Aufbringen eines Wirkstoffs auf ein Substrat, wobei der Wirkstoff in einer Mikroemulsion gelöst ist, dass die Mikroemulsion ein Aufschäummittel und optional Schaumbildner enthält, sodass sie auf einem Substrat aufschäumbar ist, und wobei Aufschäummittel und Schaumbildner so gewählt wird, dass die Teilchen der stückigen Phase, das heisst die Partikel der Mikroemulsion im wesentlichen unzerstört bleiben,sodass die Teilchen der stückigen Phase beim Zerfallen des Schaums auf dem Substrat anlagerbar sind.

2. Verfahren nach Anspruch 1 enthaltend die Schritte:
i) Bereitstellen einer Mikroemulsion enthaltend einen Wirkstoff;
ii) Bereitstellen von wenigstens einem Aufschäummittel und gegebenenfalls einem Schaumbildner, wobei Schaumbildner und Aufschäummittel im Hinblick auf die Zusammensetzung der Mikroemulsion so ausgewählt werden, dass sie im wesentlichen nicht in die Teilchen der stückigen Phase diffundieren;
iii) Bereitstellen eines Behälters zur Aufnahme der Mikroem ulsion und Aufschäummittel sowie gegebenenfalls Schaumbildner.

3. Verfahren zum Aufbringen eines Wirkstoffs mit einer Mikroemulsion auf ein Substrat enthaltend die Schritte:
i) Bereitstellen der Mikroemulsion enthaltend einen Wirkstoff;
ii) Bereitstellen von wenigstens einem Aufschäummittel und gegebenenfalls einem Schaumbildner, wobei Aufschäummittel und Schaumbildner im Hinblick auf die Zusammensetzung der Mikroemulsion so ausgewählt werden, dass sie im wesentlichen nicht in die Teilchen der stückigen Phase diffundieren;
iii) Bereitstellen eines Behälter zur Aufnahme von Mikroemulsion und Aufschäummittel, gegebenenfalls Schaumbildner, wobei der Behälter so als Schaumspender ausgebildet ist, dass die Mikroemulsion unter Ausbildung eines Schaumes auf ein Substrat auftragbar ist.

4. Verfahren zum Herstellen einer Mikroemulsion, insbesondere zur Verwendung in einem Verfahren gemäss einem der Ansprüche 1 bis 3 enthaltend die Schritte:
i) Bereitstellen eines Mikroemulsion-Prekonzentrates;
ii) Vermischen des Mikroemulsion-Prekonzentrates mit Wasser oder einem wässerigen Medium, wobei spontan die Mikroemulsion gebildet wird;
iii) Zugabe von wenigstens einem Aufschäummittel und gegebenenfalls einem Schaumbildner wobei Aufschäummittel und Schaumbildner im Hinblick auf die Zusammensetzung der Mikroemulsion so ausgewählt werden, dass sie im wesentlichen nicht in die Teilchen der stückigen Phase diffundieren.

5. Verfahren nach Anspruch 4 **dadurch gekennzeichnet, dass** der Wirkstoff in das Mikroemulsion-Prekonzentrat und/oder in die Wasserphase der Mikroemulsion eingebracht wird.

6. Zusammensetzung in Form einer Mikroemulsion zum Aufbringen eines Wirkstoffs auf ein Substrat, insbesondere Verwendung in einem Verfahren nach einem der Ansprüche 1 bis 3, enthaltend eine Mikroemulsion mit wenigstens einem Aufschäummittel und optional einem Schaumbildner.

7. Zusammensetzung nach Anspruch 6 **dadurch gekennzeichnet dass** das Aufschäummittel ein flüssiges Gas ist.

8. Zusammensetzung nach Anspruch 6 oder 7, **dadurch gekennzeichnet, dass** das Aufschäummittel einen Siedepunkt > -25°C, vorzugsweise > 0°C aufweist.

9. Zusammensetzung nach einem der Ansprüche 6 bis 8, **dadurch gekennzeichnet, dass** das Aufschäummittel Isobutan, Isopentan oder Mischungen davon enthält.

10. Zusammensetzung nach einem der Ansprüche 6 bis 9, **dadurch gekennzeichnet, dass** der Schaumbildner ausgewählt ist aus der Gruppe der Betaine, Sulfosuccinate oder Sarcosin-Derivate.

11. Zusammensetzung nach einem der Ansprüche 6 bis 10 **dadurch gekennzeichnet, dass** sie eine O/W-Mikroemulsion mit einer durchschnittlichen Teilchengrösse unter 200 nm, bevorzugt unter 150 nm, besonders bevorzugt unter 100 nm ist.

12. Zusammensetzung in Form eines Mikroemulsion-Prekonzentrates, zur Herstellung einer Mikroemulsion nach einem der Ansprüche 6 bis 11, enthaltend
(a) eine Mischung bestehend aus einem mittelkettigen Triglycerid und einer Omega-9-Fettsäure und/oder einer Omega-6-Fettsäure
(b) eine oberflächenaktive Komponente enthaltend ein Tensid vom Polyoxyethylentyp

13. Zusammensetzung nach Anspruch 12 **dadurch gekennzeichnet, dass** das mittelkettige Triglycerid Fettsäurereste mit 4-18, vorzugsweise 6-18, C-Atomen aufweist.

14. Zusammensetzung nach einem der Ansprüche 12 oder 13 **dadurch gekennzeichnet, dass** die Omega-9-Fettsäure und/oder die Omega-6-Fettsäure 12-24, insbesondere 16-24, vorzugsweise 18-22 C-Atome aufweist.

15. Zusammensetzung nach einem der Ansprüche 12 bis 14 **dadurch gekennzeichnet, dass** sie als Komponente (a) eine Mischung aus einem Capryl-/Caprinsäure-Triglycerid, Ölsäure und/oder Linolsäure enthält.

16. Zusammensetzung in Form eines Mikroemulsion-Prekonzentrats, zur Herstellung einer Mikroemulsion nach einem der Ansprüche 6 bis 11, enthaltend
(a1) ein Phospholipid
(a2) ein natürliches oder ein synthetisches oder ein partialsynthetisches Di- oder Triglycerid, sowie optional ein Wachs, einen Fettalkohol, Derviate dieser Stoffe sowie Mischungen dieser Stoffe
(b) eine oberflächenaktive Komponente enthaltend ein Tensid vom Polyoxyethylentyp.

17. Zusammensetzung nach Anspruch 16 enthaltend als Komponente (a1) ein Phospholipid, ein hydriertes oder teilhydriertes Phospholipid, ein Lysophospholipid, ein Ceramid oder Mischungen aus diesen Verbindungen 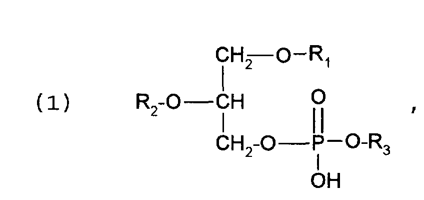 worin
R₁ C₁₀-C₂₀-Acyl;
R₂ Wasserstoff oder C₁₀-C₂₀-Acyl
R₃ Wasserstoff, 2-Trimethylamino-1-ethyl, 2-Amino-1-ethyl,
nicht substituiertes oder durch eine oder mehrere Carboxy-, Hydroxy- oder Amino-Gruppen substituiertes C₁-C₅-Alkyl; die Inositol- oder die Glycerylgruppe;
bedeuten; oder Salze dieser Verbindungen.

18. Zusammensetzung nach einem der Ansprüche 16 oder 17 enthaltend als Komponente (a2) ein mittelkettiges Triglycerid, vorzugsweise ein Capryl-/Caprinsäure Trigycerid.

19. Zusammensetzung in Form einer Mikroemulsion erhältlich durch Vermischen eines Mikroemulsion-Prekonzentrates gemäss einem der Ansprüche 12 bis 18 mit Wasser oder einem wässrigen Medium.

20. Zusammensetzung nach einem der Ansprüche 12 bis 19 enthaltend einen Wirkstoff, insbesondere einen pharmazeutischen Wirkstoff, einen biotechnologischen Wirkstoff, einen kosmetischen Wirkstoff oder einen technischen Wirkstoff.

21. Zusammensetzung zum Aufbringen eines lipophilen Wirkstoffs auf ein Substrat, wobei der Wirkstoff in den Teilchen der stückigen Phase einer Mikroemulsion gelöst ist, und wobei die Mikroemulsion ein Aufschäummittel und optional Schaumbildner enthält, so dass sie auf einem Substrat aufschäumbar ist, und wobei Aufschäummittel und Schaumbildner so gewählt sind, dass die Teilchen der stückigen Phase, das heisst die Partikel der Mikroemulsion im wesentlichen unzerstört bleiben, so dass die Teilchen beim Zerfallen des Schaums auf dem Substrat anlagerbar sind.

22. Zusammensetzung zum Aufbringen eines wasserlöslichen Wirkstoffs auf ein Substrat, wobei der Wirkstoff in der wässrigen Phase einer Mikroemulsion gelöst ist und/oder an der Grenzfläche stückige Phase/Wasser Phase an-/eingelagert ist, und wobei die Mikroemulsion ein Aufschäummittel und optional Schaumbildner enthält, so dass sie auf einem Substrat aufschäumbar ist, und wobei Aufschäummittel und Schaumbildner weiterhin so gewählt sind, dass die Teilchen der stückigen Phase, das heisst die Partikel der Mikroemulsion im Wesentlichen unzerstört bleiben, sodass die Teilchen beim Zerfallen des Schaums auf dem Substrat anlagerbar sind.

23. Behälter enthaltend eine Zusammensetzung in Form einer Mikroemulsion nach einem der Ansprüche 6 bis 11, vorzugsweise hergestellt aus einer Zusammensetzung nach einem der Ansprüche 12 bis 22.

24. Behälter enthaltend eine Zusammensetzung in Form eines Mikroemulsion-Prekonzentrates nach einem der Ansprüche 12 bis 18 oder 20 und eine wässerige Phase mit wenigstens einem Aufschäummittel.

25. Behälter nach einem der Ansprüche 21 oder 24 **dadurch gekennzeichnet, dass** der Behälter ein Sprühschaumspender, insbesondere eine Aerosolspraydose, insbesondere eine ein- oder zweikammerige Aerosolspraydose, ist.

26. Behälter nach einem der Ansprüche 21 bis 25 **dadurch gekennzeichnet dass** der Behälter wenigstens ein Treibmittel, insbesondere ein Flüssiggas oder komprimiertes Gas, vorzugsweise komprimierter Stickstoff, enthält.

27. Verwendung einer Zusammensetzung nach einem der Ansprüche 6 bis 22 zum Erzeugen eines die Mikroemulsion enthaltenden Schaums auf einem Substrat.

28. Verwendung einer Zusammensetzung nach einem der Ansprüche 6 bis 22 zum Auftragen einer Mikroemulsion mit wenigstens einem pharmazeutischen Wirkstoff in Form eines Schaums auf die Haut.

29. Verwendung einer Zusammensetzung nach einem der Ansprüche 6 bis 22 zum Auftragen einer Mikroemulsion mit wenigstens einem kosmetischen Wirkstoff in Form eines Schaums auf die Haut.

30. Verwendung einer Zusammensetzung nach einem der Ansprüche 6 bis 22 zum Auftragen einer Mikroemulsion mit wenigstens einem biotechnologischen Wirkstoff, insbesondere einem Protein oder einem Peptid, in Form eines Schaums auf ein Substrat.

31. Verwendung einer Zusammensetzung nach einem der Ansprüche 6 bis 22 zum Auftragen einer Mikroemulsion mit wenigstens einem technischen Wirkstoff in Form eines Schaums auf ein Substrat.

32. Verwendung eines ein- oder zweikammerigen Schaumspendebehälters zum Auftragen einer nach dem Auftragen aufschäumenden Mikroemulsion nach einem der Ansprüche 6 bis 22.

33. Verwendung nach einem der Ansprüche 27 bis 32 zur topischen Applikation von Dexpanthenol.
